# EUROPEAN PATENT SPECIFICATION

(11) **EP 4 282 336 B1**
(45) Date of publication and mention of the grant of the patent: **03.06.2026**
(21) Application number: 22742016.3
(22) Date of filing: 05.01.2022
(51) Int. Cl.: A61B 6/00, G06T 3/40

(54) **SCANNING IMAGING SYSTEM AND METHOD BASED ON SLIT X-RAYS**
ABTASTENDES ABBILDUNGSSYSTEM UND VERFAHREN AUF DER BASIS VON SPALTRÖNTGENSTRAHLEN
SYSTÈME ET PROCÉDÉ D'IMAGERIE À BALAYAGE BASÉS SUR DES RAYONS X FENDUS

(30) Priority: 22.01.2021 CN 202110093581; 22.01.2021 CN 202120181258 U
(43) Date of publication of application: 29.11.2023
(73) Proprietor: Shanghai Taoimage Medical Technology Co., Ltd., Shanghai 201210 (CN)
(72) Inventor: TSAI, Tsungyuan, Shanghai 201204 (CN); DAI, Xingwu, Shanghai 201204 (CN); ZHONG, Jinbing, Shanghai 201204 (CN)
(74) Representative: Argyma
(86) International application number: PCT/CN2022/070326
(87) International publication number: WO 2022/156537

(56) References cited:
- WO-A1-2018/096208
- CN-A- 1 684 629
- CN-A- 102 124 320
- CN-A- 105 405 101
- CN-Y- 2 879 977
- US-A1- 2010 177 948
- US-A1- 2013 058 459
- US-A1- 2020 029 916
- TAM�S ILL�S ET AL: "The EOS(TM) imaging system and its uses in daily orthopaedic practice", INTERNATIONAL ORTHOPAEDICS, SPRINGER, BERLIN, DE, vol. 36, no. 7, 28 February 2012 (2012-02-28), pages 1325 - 1331, XP035075705, ISSN: 1432-5195, DOI: 10.1007/S00264-012-1512-Y

## Description

### Field of the Invention

The present invention relates to the technical field of digital X-ray imaging methods, in particular to a slit X-ray based scanning imaging system and method.

### Description of the Prior Art

At present, the medical imaging technology has been widely used in clinical practice. When detecting orthopedic deformities, especially those of the spine and lower limbs, it is necessary to take complete full-length radiographs of complete images of the spine and lower limbs to visually display deformities and abnormal limb force lines, and like situations in order to reflect the true limb force lines. In the prior art, a common X-ray scanning imaging system configured for shooting an orthopedic full-length radiograph is that an X-ray is emitted by an emission end and an image of an object to be imaged at each moving step is collected by a detection end, and the full-length radiograph is obtained by performing stitching through an image similarity algorithm or a feature point matching algorithm in an overlapping region of two adjacent images; the image quality of the full-length radiograph obtained by such a scanning imaging system has a severe distortion; and with the development of slit radiography X-ray technology, the slit X-ray based scanning imaging system can reduce the coincidence degree of an edge position for imaging. However, the edges of each image formed by image stitching, especially the overlapping regions with a high similarity, may have spatial distortion.

It is known by US2020/029916A1 a method for operating an X-ray tube assembly wherein a collimator with adjustable aperture enables to acquire depth information. It is known by US2010/177948A1 a method enabling to determine a magnification factor associated with an image wherein a front image and a lateral image are acquired.

Based on the above technical problems, there is an urgent need for a slit X-ray based scanning imaging system and method to achieve more accurate image position positioning and image stitching.

### SUMMARY OF THE INVENTION

In view of the above-mentioned problems, the object of the present invention is to provide a slit X-ray based scanning imaging system and method. The X-ray is collimated by means of slit scanning, the slit X-ray of a predetermined width is emitted in a ray emission window, the ranges of emission and reception at any moment of the ray emission window and the slit scanning region of interest are controlled to be consistent by a position control module, the received original images and stitching parameters of the original images are stored, the received stitching parameters of the original images serve as addressing information, and the received original images are reconstructed according to the stitching parameters of the scanning images. A complete image of the object to be imaged is stitched.

In order to achieve the above object, a first aspect of the present invention provides a slit X-ray based scanning imaging system, as defined in claim 1.

In an implementation mode of the present application, the scanning imaging system further comprises: a system initialization module for initializing a scanning imaging system, further comprising:
a mode acquisition unit configured for acquiring an imaging data set of the object to be imaged;
a parameter passing unit configured for passing the imaging data set to the scanning imaging system; and
an initial position response unit configured for controlling, in response to the imaging data set, the ray emission window and the preset slit region of interest to be respectively located in a system initial position.

Further, the position calibration unit of the system of claim 1 comprises:
an initial position calibration sub-unit comprising a first optical indication device pointing to the initial position of the object to be imaged along the ray emission window and configured for positioning and calibrating the initial position of the object to be imaged pointed to by the ray emission window and the preset slit region of interest;
a tail end position calibration sub-unit comprising a second optical indication device configured for generating an indication signal pointing to a tail end position of the object to be imaged, and performing positioning and calibrating on the tail end position of the object to be imaged pointed to by the ray emission window and the preset slit region of interest according to the indication signal; and
a relative position calibration sub-unit comprising a position adjustment device configured for positioning and calibrating relative positions of the ray emission window and the preset slit region of interest.

Further, the position synchronization unit and the position calibration unit are responsive to the position tracking unit;
the position tracking unit is configured to track positions of the ray emission window and the preset slit region of interest by means of spatial coordinate position information.

In an implementation mode of the present application, an emission module of a scanning imaging system comprises:
a collimation unit configured for collimating the X-ray to have the width of the ray emission window; and
an emission control unit configured for controlling an emission interval of the X-ray.

In an implementation mode of the present application, the imaging module of the scanning imaging system further comprises:
an original image storage sub-module configured for storing the original images of the object to be imaged and the stitching parameters of the original images received by the preset slit region of interest in the case where the preset slit region of interest receives the X-ray attenuated at any position of the object to be imaged.

An original image transmission sub-module is configured for transmitting the original images generated by the preset slit region of interest and the stitching parameters of the original images.

Further, the original image storage sub-module further comprises:
a ray receiving unit configured for receiving the X-rays attenuated after passing through the object to be imaged;
an image generation unit configured for generating the original images of the object to be imaged corresponding to attenuated X-rays; and
an image marking unit configured for marking the stitching parameters of the original images.

Further, the original image transmission sub-module further includes:
an original image collection unit configured for collecting the original images of the image storage sub-module and stitching parameters of the original images according to position trajectories of the ray emission window and the preset slit region of interest; and
an original image output unit configured for outputting the original images and the stitching parameters of the original images collected by the original image collection unit to the image reconstruction module.

In an implementation mode of the present application, an image reconstruction module of a scanning imaging system is configured to reconstruct the original images according to the stitching parameters output by the image output unit to form a complete image in the case where the image output unit outputs the original images at the tail end position of the object to be imaged, wherein the image reconstruction module comprises:
an addressing unit configured for searching for the original image to be reconstructed based on the stitching parameters of the original images that can be addressed one by one; and
a stitching unit configured for adjusting, according to the stitching parameters, a stitching of a tail end position of a previous imaging and a beginning position of the next imaging of the original images to be reconstructed to form a complete image.

A second aspect of the present application provides a slit X-ray based scanning imaging as defined in claim 10.

Compared to the prior art, the advantageous effects of the present invention are as follows.

The present application provides a slit X-ray based scanning imaging system and method. Parameter information about the scanning imaging system is preset via a system initialization module, the X-ray is collimated by means of slit scanning, the slit X-ray of a predetermined width is emitted in a ray emission window, the ranges of the emitted and received regions at any moment of the ray emission window and the slit scanning region of interest are controlled to be consistent via a position control module, and the received original image and original image stitching parameters thereof are stored by an original image storage sub-module. The image and the stitching parameters of the original image are transmitted via the image transmission sub-module, and the image stitching parameters serve as addressing information. According to the stitching parameters of the image, the image is reconstructed and stitched into a complete image of an object to be imaged. The above-mentioned technical solution emits X-rays through a slit, and realizes that the ranges of emission and reception at any position of the ray emission window and the slit scanning region of interest are completely consistent through a position control module, so as to ensure that the imaging range is clear under the condition that the amount of X-ray radiation quantity received by an object to be imaged is as small as possible; at the same time, the image reconstruction and stitching are realized by acquiring the stitching parameters of the original image, and the accurate control of the imaging position and the acquisition of the stitching parameters of the imaging image are realized, so that the object to be imaged can greatly weaken the coincidence degree of the edge position of the imaging under little radiation dose, and at the same time, the spatial distortion existing in the overlapping regions with a high similarity is reduced to form a complete image with high quality.

### BRIEF DESCRIPTION OF THE DRAWINGS

Various other advantages and benefits will become apparent to those of ordinary skill in the art upon reading the following detailed description of preferred implementation modes. The drawings are only for the purposes of illustrating the preferred implementation modes and are not to be construed as limiting the invention.
Fig. 1 shows a schematic block diagram of a slit X-ray based scanning imaging system;
Fig. 2 is a schematic diagram showing a structure for controlling the consistency of the positions of a ray emission window and a preset slit region of interest according to one embodiment of the present invention;
Fig. 3 is a schematic diagram showing a structure for controlling the consistency of the positions of the ray emission window and the preset slit region of interest according to another embodiment of the present invention;
Fig. 4 shows a schematic diagram for implementing image stitching and reconstructing based on taking an overlapping region of adjacent images as a stitching parameter; and
Fig. 5 shows a flow chart of a slit X-ray based scanning imaging method.

### DESCRIPTION OF THE PREFERRED EMBODIMENTS

Unless defined otherwise, technical or scientific terms used in the description and claims shall have the ordinary meaning as understood by one of ordinary skill in the art to which the present invention belongs.

In describing the present invention, it needs to be understood that the orientations or positional relationships indicated by the terms "center", "longitudinal", "transverse", "up", "down", "front", "rear", "left", "right", "vertical", "horizontal", "top", "bottom", "inner", "outer" and the like are based on the orientations or positional relationships shown in the drawings for purposes of describing the present invention and simplifying the description only, and are not intended to indicate or imply that the referenced device or element must have a particular orientation or be constructed and operated in a particular orientation. It is therefore not to be understood as limiting the present invention.

It will be understood by those skilled in the art that, as used herein, the singular forms "a", "one" and "the" may include the plural forms as well, unless expressly stated otherwise. It should be further understood that the word "comprises/comprising" when used in this description of the invention is taken to specify the presence of stated features, integers, steps, operations, elements, and/or assemblies, but does not preclude the presence or addition of one or more other features, integers, steps, operations, elements, assemblies, and/or groups thereof.

Hereinafter, the present invention will be described in detail in conjunction with the embodiments with reference to the accompanying drawings. It needs to be pointed out that, in the detailed description of the implementation modes, in the interest of brevity and conciseness, not all features of actual implementation modes may be described in the description.

According to the slit X-ray based scanning imaging system and method provided by the present invention, the initialization parameters of the scanning imaging system set according to the imaging mode are used, the ray emission window and the preset slit region of interest are controlled to emit and receive completely consistent at any position via the initialization parameters, the whole original images of the object to be imaged are generated, and the original images are reconstructed to form a complete image of the object to be imaged according to the stitching parameters of the original images. The following are explanations through specific embodiments.

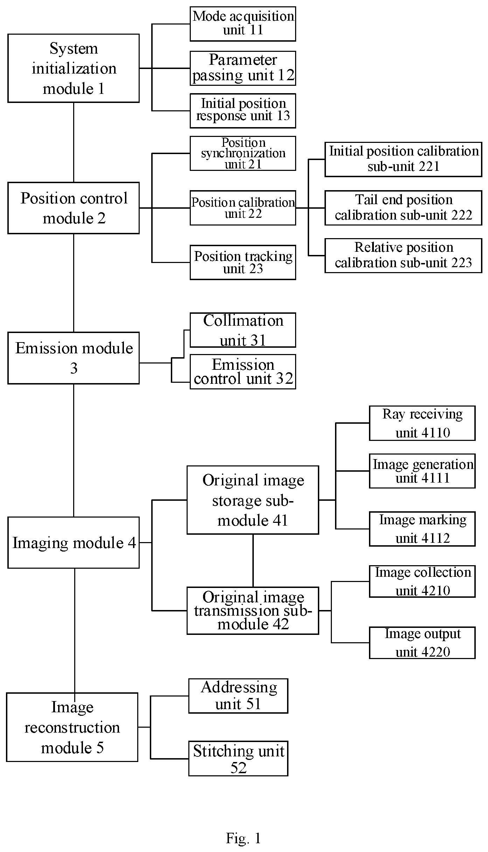

As shown in Fig. 1, this embodiment provides a schematic block diagram of a slit X-ray based scanning imaging system, specifically including: a system initialization module 1 configured for initializing the scanning imaging system before the scanning imaging starts and/or after the scanning imaging ends.

Specifically, before the scanning imaging system works, initialization parameters can be set according to requirements including an imaging position, imaging information display, imaging type, etc. of an object to be imaged. The initialization parameters include a preset scanning position parameter and a preset scanning frequency.

Specifically, when the scanning imaging system is performed on an object to be imaged to form a complete scanning, it is necessary to set the scanning system to restore the state of to be scanned and imaged so as to receive system initialization parameter information at the next scanning.

It needs to be noted that the imaging type of the scanning imaging system can be determined according to the position and property of the actual object to be imaged, and the imaging type may change when the object to be imaged changes, which is not limited herein.

Specifically, in case the imaging type of the object to be imaged is determined, the system initialization module 1 includes:
a mode acquisition unit 11 configured for acquiring an imaging data set of the object to be imaged;
a parameter passing unit 12 configured for passing the imaging data set to the scanning imaging system; and
an initial position response unit 13 configured for controlling, in response to the imaging data set, the ray emission window and the preset slit region of interest to be respectively located in a system initial position.

Specifically, in this step, the imaging data set includes information that can include system initial data, such as scanning position parameter, scanning frequency parameter, a one-time scanning region of the object to be imaged, image denoising parameter, enhancement parameter, scanning image reconstruction parameter, etc.; the imaging data set is passed to the scanning imaging system; the scanning imaging system controls, according to the imaging data set, the size of the ray emission window and the size of the preset slit region of interest to be completely the same. Close to the initial imaging position of the object to be imaged, the ray emission window and the preset slit region of interest are at relative positions of the same central horizontal line.

Specifically, the scanning imaging system further comprises:
a position control module 2 configured for controlling X-rays emitted by the ray emission window at any position to be exactly and completely received by the preset slit region of interest; Specifically, the position control module 2 receives scanning frequency information in the initialization information of the scanning imaging system, and control the range of the rays emitted and received by the ray emission window and the preset slit region of interest to be consistent in the case where the scanning frequencies are the same. The position control module 2 includes:
a position synchronization unit 21 comprising a controller configured for controlling the positions of the ray emission window and the preset slit region of interest;
and a position calibration unit 22 configured for positioning and calibrating the ray emission window and the preset slit region of interest.

The position calibration unit 22 includes: an initial position calibration sub-unit 221 comprising a first optical indication device pointing to the initial position of the object to be imaged along the ray emission window and configured for positioning and calibrating the initial position of the object to be imaged pointed to by the ray emission window and the preset slit region of interest;
a tail end position calibration sub-unit 222 comprising a second optical indication device configured for generating an indication signal pointing to a tail end position of the object to be imaged, and performing positioning and calibrating on the tail end position of the object to be imaged pointed to by the ray emission window and the preset slit region of interest according to the indication signal; and
a relative position calibration sub-unit 223 comprising a position adjustment device configured for positioning and calibrating relative positions of the ray emission window and the preset slit region of interest.

The position control module 2 further includes a position tracking unit 23 configured for updating positions of the ray emission window and the preset slit region of interest.

The position tracking unit 23 is configured to track the positions of the ray emission window and the preset slit region of interest by means of spatial coordinate position information.

**In** some embodiments of the present application, the controller enables the scanning imaging system to synchronously move the positions of the ray emission window and the preset slit region of interest at any time period from the completion of the initialization to the scanning imaging process of the object to be imaged. The ray emission window and the preset slit region of interest can be kept always synchronously moved by a servo motor, a stepper motor, etc. without being limited herein.

**In** some embodiments of the present application, the first optical indication device is directed to a starting position of the object to be imaged, the position of the slit emission window movement determines that the starting position of the object to be imaged is exactly within the imaging field of view, and the imaging scanning starting position of the scanning imaging system is determined. The second optical indication device can set an optical indication lamp via a movable sliding block, pre-align with the scanning tail end position of an object to be imaged, and pass an optical signal aligned with the tail end position as an indication signal to a data reading module of the scanning imaging system via a serial communication port. The serial communication port can read the distance from the tail end position to a certain horizontal plane, such as the ground, as a scanning tail end position according to indication signal information, and pass the same to the position control module 2 of the scanning imaging system to determine the tail end position of the scanning imaging. When the scanning of the scanning imaging system is completed at the tail end position, the scanning is completed.

Specifically, the first optical indication device generates the scanning staring signal and the second optical indication device generates an end signal corresponding to the scanning tail end so as to realize accurate positioning of an initial position and an ending position of scanning imaging of an object to be imaged, and may include a movable sliding block, an optical indication lamp, a serial communication module, etc. which are not limited herein.

In some embodiments of the present application, the position adjustment device receives the stitching parameter of the information about the position tracking unit 23, and makes an adaptive adjustment according to the relative positions of the ray emission window and the preset slit region of interest, so as to achieve the complete consistency of the emitted region and the received region. The position adjustment device can be implemented by a mechanical arm positioning device, a driving screw orientation displacement device, etc.

Specifically, the scanning imaging system further comprises:
an emission module 3 configured for emitting X-rays having the width of the ray emission window to an object to be imaged in the case where the ray emission window and the preset slit region of interest are located at an initial position of the object to be imaged. The emission module 3 includes:
a collimation unit 31 configured for collimating the X-ray to have the width of the ray emission window; and
an emission control unit 32 configured for controlling an emission interval of the X-ray.

Specifically, the X-ray emitted by the width of the ray emission window is a slit X-ray which is collimated by the collimation unit such that the size of the collimated slit X-ray is the same as the size of the preset slit region of interest; and the collimation unit can be implemented by an automatic control light concentrator arranged at the emission end of the scanning emission system.

Specifically, controlling the emission interval of the X-rays according to the scanning frequency achieves the same frequency as the movement of the positions of the ray emission window and the preset slit region of interest.

Specifically, the scanning imaging system comprises an imaging module 4 and an image reconstruction module 5.

The imaging module 4 is configured for generating original images based on received X-rays passing through the object to be imaged, wherein at least an overlapping region exists between adjacent images of the original images. It can be understood that when the X-ray passes through the object to be imaged, original images with a certain degree of difference can be formed based on the basic characteristics of the X-ray, namely, penetrability, absorbability, fluorescent effect, photosensitive effect, and the inherent properties of the structure of the object to be imaged; and by setting the initialization parameters of the system, and by at least one overlapping region existing between adjacent original images, it is ensured that no breakpoint appears in the image formation, and the images of the object to be imaged can be displayed completely.

The imaging module 4 further comprises an original image storage sub-module 41 and an original image transmission sub-module 42.

The original image storage sub-module 41 is configured for storing the original images of the object to be imaged and the stitching parameters of the original images received by the preset slit region of interest in the case where the preset slit region of interest receives the X-ray attenuated at any position of the object to be imaged. The original image storage sub-module 41 includes:
a ray receiving unit 4110 configured for receiving the X-rays attenuated after passing through the object to be imaged;
an image generation unit 4111 configured for generating the images of the object to be imaged corresponding to attenuated X-rays; and
an image marking unit 4112 configured for marking the stitching parameters of the images.

Specifically, the ray emission window emits a slit X-ray, and the preset slit region of interest automatically activates and receives a slit X-ray with a certain attenuation formed after passing through an object to be imaged, generates a scanning imaging image at a corresponding position of each X-ray emission region according to scanning data and according to preset initialization information received by the scanning imaging system, and marks a stitching parameter of the image, namely, information for marking an image with stitching points, including spatial position information about the image, initial position information about the imaging of an original image, and position information at the end, as addressing information about image stitching.

The original image transmission sub-module 42 is configured for transmitting the original images and the stitching parameter of the original images generated by the preset slit region of interest. The original image transmission sub-module 42 includes:
an image collection unit 4210 configured for collecting the original images of the image storage sub-module 41 and stitching parameters of the original images according to position trajectories of the ray emission window and the preset slit region of interest; and
an image output unit 4220 configured for outputting the original images and the stitching parameters of the original images collected by the image collection unit to the image reconstruction module 5.

Specifically, after receiving the generated image, the image collection unit is triggered to perform image collection, and stitching parameters of the collected images are saved, and the triggering can be realized by means of an infrared sensor or a loop formed by an electrical connection, etc. which is not limited herein.

The image reconstruction module 5 is configured for reconstructing the original images to form a complete image according to stitching parameters of the original images, wherein the stitching parameters at least comprise stitching point marking information about the original images. The following is further included:
an addressing unit 51 configured for searching for the original images to be reconstructed based on the stitching parameters of the original images that can be addressed one by one; and
a stitching unit 52 configured for adjusting, according to the stitching parameters, a stitching of a tail end position of a previous imaging and a beginning position of the next imaging of the original images to be reconstructed to form a complete image.

It needs to be noted that the stitching parameter of the generated image can be used as one image tag to correspond to the imaging of each scanning imaging region on a one-to-one basis. According to the starting position and the tail end position of the object to be imaged and according to the stitching parameter information, the tail end of the previous image and the starting end of the next image of adjacent original images are adjusted to be connected, and all the images to be reconstructed are successively stitched.

Specifically, when the scanning imaging object is located at the initial position of the object to be imaged, the mark for the image stitching parameter points to the tail end imaging position of the first original image. For the tail end position of the object to be imaged, the mark for the image stitching parameter points to the starting end position of the last original image. At any intermediate position, the mark needs to point to the starting end position and the tail end position of the original image so as to achieve complete stitching and reconstruction of all original images from the starting position to the tail end position. The specific implementation methods for image stitching will be explained in detail below.

Specifically, the images to be reconstructed by sequential stitching can be reconstructed by only using the image stitching parameter, the image stitching parameter can be acquired based on overlapping region information about each original image, and the image quality can be ensured for the stitching parameter acquired by using the overlapping region information. For more complex images, it is also possible to further optimize the reconstructed image on the basis of stitching parameters in combination with algorithms such as feature point matching and adjacent point searching of the original image, which is not limited herein.

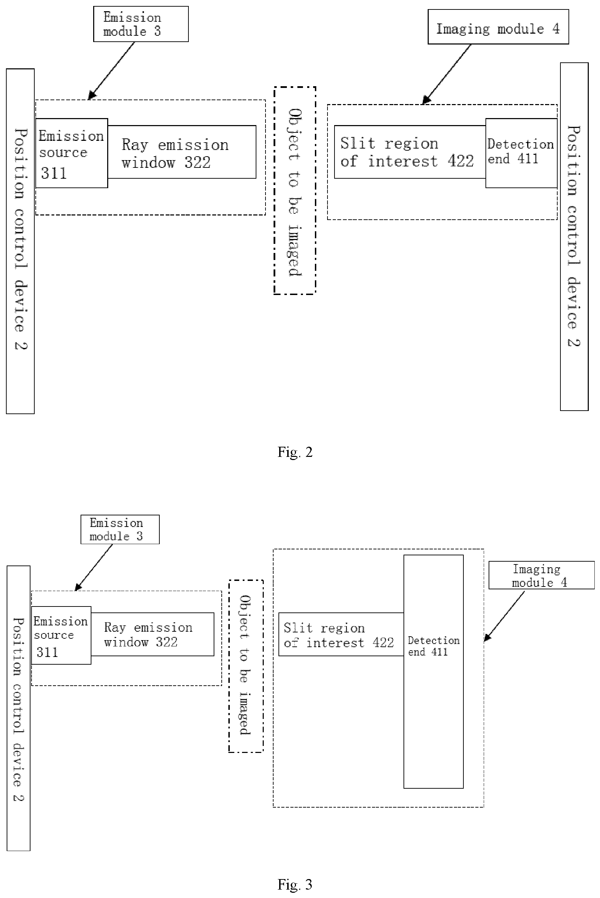

As shown in Fig. 2, the present embodiment shows a schematic structural diagram for controlling the positions of the ray emission window and the preset slit region of interest to be consistent, and specifically comprises the following.

The position control module 2 simultaneously controls the synchronous movement of the emission module 3 and the imaging module 4; the detection end 411 of the imaging module 4 is set as a short-length flat panel detector; the preset slit region of interest moves with the flat panel detector; and the movement position of the emission source 311 is consistent with the flat panel detector of the detection end 411, thereby achieving the consistency of the positions of the ray emission window 322 and the preset slit region of interest 422.

As shown in Fig. 3, the present embodiment shows a structural diagram for keeping the positions of a ray emission window and a preset slit region of interest consistent, and specifically comprises the following.

The position control module 2 controls the movement of the emission module 3; the detection end 411 of the imaging module 4 is set as a long-length flat panel detector; during the process of the scanning imaging system scanning the object to be imaged for imaging, the detection end 411 does not need to move; the preset slit region of interest 422 is automatically activated according to the slit X-ray emitted by the ray emission window; and the movement position of the emission source 311 is consistent with the preset slit region of interest 422, thereby achieving the consistency of the positions of the ray emission window and the preset slit region of interest.

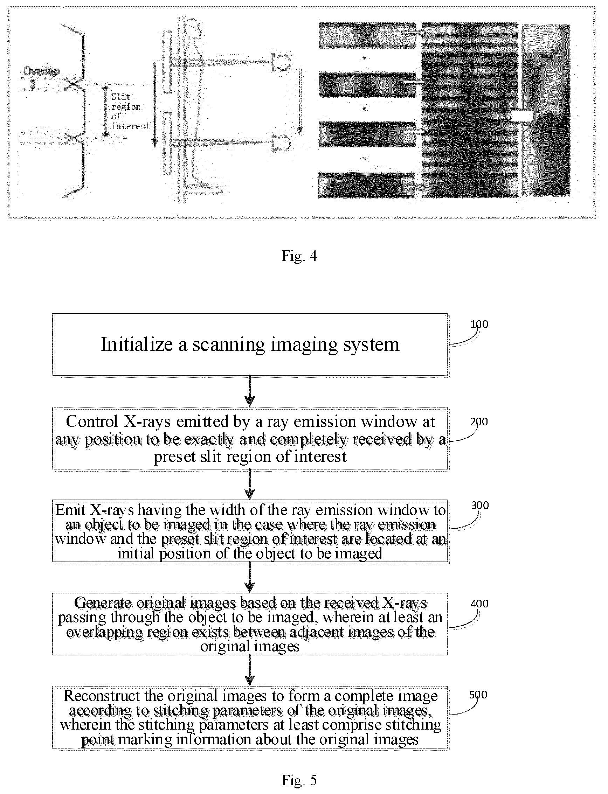

As shown in Fig. 4, the present embodiment shows a schematic diagram for implementing image stitching and reconstructing based on taking an overlapping region of adjacent images as a stitching parameter, and specifically comprises the following.

Original images of X-ray imaging from top to bottom are successively acquired; when the original images are stored, a method for successively placing images from top to bottom for model imaging can be used, and the initialization parameter information of the scanning imaging system can be configured to acquire the spatial position information of the current original image imaging recorded by the scanning imaging system every time the original image is formed; the spacing Displacement between adjacent images is acquired based on the position information of the current original image; in the case where the Displacement is determined, the overlapping region Overlap of the adjacent original images can be acquired according to the initialization parameter information about the scanning imaging system, including the width of the slit region of interest SlotSize, the pixel spacing Pixelpitch of a detection module, the spacing SID between the focus point of the emission source and the imaging plane of the detection module, and the preset spacing OID between the object to be imaged and the imaging plane of the detection module; and the overlapping region forms an Overlap numerical value so as to ensure that when the spacing OID between the object to be imaged and the imaging plane of the detection module is a numerical value of any scannable imaging during any shooting of an image, the overlapping region Overlap is present. When the scanning imaging system scans and images the object to be imaged, there may be a certain deviation between the spacing OID between the object to be imaged and the imaging plane of the detection module, and the preset value. At this moment, the overlapping region Overlap is calculated according to the actual OID value, and the specific calculation formula may be:
According to the overlapping region Overlap as the stitching parameter information of the original image, the original images are stitched and reconstructed in a certain order to form a complete imaging image of the object to be imaged.

In some embodiments of the present application, when the scanning speed of the scanning imaging system for imaging the object to be imaged changes, the Displacement changes as well. Each time the original image is stitched, the Overlap needs to be recalculated to acquire new stitching parameter information.

Specifically, the scanning speed of the scanning imaging system for imaging the object to be imaged can be correspondingly changed according to the property of the object to be imaged. For example, the scanning speed can be increased in the case where the scanning area required for one scanning of the object to be imaged itself is small, so that the object to be imaged can obtain a completed image satisfying the requirements of the imaging image in the case where the object to be imaged receives little ray radiation. The specific setting of scanning parameters can be based on the imaging characteristics of the object to be imaged, which is not limited herein.

As shown in Fig. 5, this embodiment shows a flow chart of a slit X-ray based scanning imaging method, specifically comprising:
step 100: initializing a scanning imaging system;
step 200: controlling X-rays emitted by a ray emission window at any position to be exactly and completely received by a preset slit region of interest;
step 300: emitting X-rays having the width of the ray emission window to an object to be imaged in the case where the ray emission window and the preset slit region of interest are located at an initial position of the object to be imaged;
step 400: generating original images based on the received X-rays passing through the object to be imaged, wherein at least an overlapping region exists between adjacent images of the original images; and
step 500: reconstructing the original images to form a complete image according to stitching parameters of the original images, wherein the stitching parameters at least comprise stitching point marking information about the original images.

Specifically, the slit X-ray based scanning imaging method described above can be implemented by the scanning imaging system as in the foregoing embodiments, which will not be described in detail herein.

In summary, the present application provides a slit X-ray based scanning imaging system and method. Parameter information about the scanning imaging system is preset via a system initialization module, the X-ray is collimated by means of slit scanning, the slit X-ray of a predetermined width is emitted in a ray emission window, the ranges of the emitted and received regions at any moment of the ray emission window and the slit region of interest are controlled to be consistent via a position control module, and the received original images and original image stitching parameters thereof are stored by an original image storage sub-module. The original images and the stitching parameters of the original images are transmitted via the image transmission sub-module, and the image stitching parameters serve as addressing information. According to the stitching parameters of the original images, the original images are reconstructed and stitched into a complete image of an object to be imaged. The above-mentioned slit X-ray based scanning image system emits X-rays through a slit, and realizes that the ranges of emission and reception at any position of the ray emission window and the slit region of interest are completely consistent through a position control module, so as to ensure that the imaging range is clear under the condition that the amount of X-ray radiation quantity received by an object to be imaged is as small as possible; at the same time, the image reconstruction and stitching are realized by acquiring the stitching parameters of the original image, and the accurate control of the imaging position and the acquisition of the stitching parameters of the imaging image are realized, so that the object to be imaged can greatly weaken the coincidence degree of the edge position of the imaging under little radiation dose, and at the same time, the spatial distortion existing in the overlapping regions with a high similarity is reduced to form a complete image with high quality.

The functional units in the embodiments of the invention may be integrated into one processing unit or each unit may be an independent physical module.

The software program of the present invention may be executed by a processor to implement the steps or functions described above. As such, the software programs (including associated data structures) of the present invention may be stored on a computer-readable recording medium, such as RAM memory, magnetic or optical drivers or floppy disks, and the like. In addition, some of the steps or functions of the present invention may be implemented in hardware, for example, as a circuit that cooperates with a processor to execute various functions or steps. The methods disclosed in the embodiments shown in the embodiments of the description can be applied to or implemented by a processor. The processor may be an integrated circuit chip having signal processing capabilities. In implementation, the steps of the methods described above may be completed by integrated logic circuits in hardware or instructions in software in a processor. The processor 101 may be a general-purpose processor, including a Central Processing Unit (CPU), a Network Processor (NP), etc.; and may also be a Digital Signal Processor (DSP), an Application Specific Integrated Circuit (ASIC), a Field-Programmable Gate Array (FPGA) or other programmable logic devices, discrete gates or transistor logic devices, discrete hardware assemblies. The various methods, steps, and logic blocks disclosed in the embodiments described herein may be implemented or executed. A general-purpose processor may be a microprocessor, but in the alternative, the processor may be any conventional processor, etc. The steps of the method disclosed in connection with the embodiments disclosed herein may be embodied directly in hardware decoding processor execution, or in an execution of a combination of hardware and software modules within the decoding processor. A software module may be located in a random access memory, a flash memory, a read-only memory, a programmable read-only memory, or an electrically erasable programmable memory, a register, and like storage media well known in the art. The storage medium is located in a memory. The processor reads the information in the memory and in combination with the hardware thereof, completes the steps of the above-mentioned method.

## Claims

1. An X-ray based scanning imaging system comprising:
an emission module (3) configured for emitting X-rays having a width of a ray emission window to an object to be imaged ;
an imaging module (4) configured for generating original images based on received X-rays passing through an object to be imaged, wherein at least an overlapping region exists between adjacent images of the original images; and
an image reconstruction module (5) configured for reconstructing the original images to form a complete image according to stitching parameters of the original images, wherein the stitching parameters at least comprise stitching point marking information about the original images,
the X-ray based scanning imaging system being **characterized in** being a slit X-ray based scanning imaging system using slit radiography X-ray technology and comprising :
a position control module (2) configured for controlling X-rays emitted by a ray emission window (322) at any position to be exactly and completely received by a preset slit region of interest (422);
the ray emission window (322) and the preset slit region of interest (422) being located at an initial position of the object to be imaged;
wherein the position control module (2) comprises:
a position synchronization unit (21) comprising a controller configured for controlling positions of the ray emission window (322) and the preset slit region of interest (422);
a position calibration unit (22) configured for positioning and calibrating the ray emission window (322) and the preset slit region of interest (422); and
a position tracking unit (23) configured for updating positions of the ray emission window (322) and the preset slit region of interest (422);
the position synchronization unit (21) and the position calibration unit (22) being responsive to the position tracking unit (23).

2. The slit X-ray based scanning imaging system according to claim 1, further comprising: a system initialization module (1) configured to initialize the scanning imaging system, wherein the system initialization module (1) comprises:
a mode acquisition unit (11) configured for acquiring an imaging data set of the object to be imaged;
a parameter passing unit (12) configured for passing the imaging data set to the scanning imaging system; and
an initial position response unit (13) configured for controlling, in response to the imaging data set, the ray emission window (322) and the preset slit region of interest (422) to be respectively located in a system initial position.

3. The slit X-ray based scanning imaging system according to claim 1, wherein the position calibration unit (22) comprises:
an initial position calibration sub-unit (221) comprising a first optical indication device pointing to the initial position of the object to be imaged along the ray emission window (322) and configured for positioning and calibrating the initial position of the object to be imaged pointed to by the ray emission window (322) and the preset slit region of interest (422);
a tail end position calibration sub-unit (222) comprising a second optical indication device configured for generating an indication signal pointing to a tail end position of the object to be imaged, and performing positioning and calibrating on the tail end position of the object to be imaged pointed to by the ray emission window (322) and the preset slit region of interest (422) according to the indication signal; and
a relative position calibration sub-unit (223) comprising a position adjustment device configured for positioning and calibrating relative positions of the ray emission window (322) and the preset slit region of interest (422).

4. The slit X-ray based scanning imaging system according to claim 3, wherein the position tracking unit (23) is configured to track positions of the ray emission window (322) and the preset slit region of interest (422) by means of spatial coordinate position information.

5. The slit X-ray based scanning imaging system according to claim 1, wherein the emission module (3) comprises:
a collimation unit (31) configured for collimating the X-rays to have the width of the ray emission window (322); and
an emission control unit (32) configured for controlling an emission interval of the X-rays.

6. The slit X-ray based scanning imaging system according to claim 1, wherein the imaging module (4) further comprises:
an original image storage sub-module (41) configured for storing the original images of the object to be imaged and the stitching parameters of the original images received by the preset slit region of interest (422) in the case where the preset slit region of interest (422) receives the X-ray attenuated at any position of the object to be imaged; and
an original image transmission sub-module (42) configured for transmitting the original images generated by the preset slit region of interest (422) and the stitching parameters of the original images.

7. The slit X-ray based scanning imaging system according to claim 6, wherein the original image storage sub-module (41) further comprises:
a ray receiving unit (4110) configured for receiving the X-rays attenuated after passing through the object to be imaged;
an image generation unit (4111) configured for generating the original images of the object to be imaged corresponding to attenuated X-rays; and
an image marking unit (4112) configured for marking the stitching parameters of the original images.

8. The slit X-ray based scanning imaging system according to claim 7, wherein the original image transmission sub-module (42) further comprises:
an original image collection unit (4210) configured for collecting the original images of the image storage sub-module (41) and the stitching parameters of the original images according to position trajectories of the ray emission window (322) and the preset slit region of interest (422); and
an original image output unit (4220) configured for outputting the original images and the stitching parameters of the original images collected by the original image collection unit (4210) to the image reconstruction module (5).

9. The slit X-ray based scanning imaging system according to claim 8, wherein the image reconstruction module (5) is configured to reconstruct the original images according to the stitching parameters output by the image output unit (4220) to form a complete image in the case where the image output unit (4220) outputs the original images at the tail end position of the object to be imaged, wherein the image reconstruction module (5) comprises:
an addressing unit (51) configured for searching for the original images to be reconstructed based on the stitching parameters of the original images that can be addressed one by one; and
a stitching unit (52) configured for adjusting, according to the stitching parameters, a stitching of a tail end position of a previous imaging and a beginning position of a next imaging of the original images to be reconstructed to form a complete image.

10. An X-ray based scanning imaging method comprising:
emitting (300) X-rays having a width of a ray emission window (322) to an object to be imaged in the case;
generating (400) original images based on received X-rays passing through the object to be imaged, wherein at least an overlapping region exists between adjacent images of the original images; and
reconstructing (500) the original images to form a complete image according to stitching parameters of the original images, wherein the stitching parameters at least comprise stitching point marking information about the original images;
the X-ray based scanning imaging method being **characterized in** being a slit X-ray based scanning imaging method using slit radiography X-ray technology and comprising :
controlling (200) X-rays emitted by the ray emission window (322) at any position to be exactly and completely received by a preset slit region of interest (422);
the ray emission window (322) and the preset slit region of interest (422) being located at an initial position of the object to be imaged;
wherein the step of controlling (200) comprises :
controlling positions of the ray emission window (322) and the preset slit region of interest (422);
positioning and calibrating the ray emission window (322) and the preset slit region of interest (422);
updating positions of the ray emission window (322) and the preset slit region of interest (422);
the position synchronization unit (21) and the position calibration unit (22) being responsive to the position tracking unit (23).

## Patentansprüche

1. Röntgenbasiertes abtastendes Abbildungssystem umfassend:
ein Emissionsmodul (3), das zum Emittieren von Röntgenstrahlen mit einer Breite eines Strahlemissionsfensters zu einem abzubildenden Objekt ausgebildet ist,
ein Abbildungsmodul (4), das zum Erzeugen von Ursprungsbildern basierend auf empfangenen Röntgenstrahlen, die ein abzubildendes Objekt durchdringen, ausgebildet ist, wobei wenigstens ein überlappender Bereich zwischen benachbarten Bildern der Ursprungsbilder vorhanden ist; und
ein Bildrekonstruktionsmodul (5), das zum Rekonstruieren der Ursprungsbilder, um gemäß Zusammenfügungsparametern der Ursprungsbilder ein vollständiges Bild zu bilden, ausgebildet ist, wobei die Zusammenfügungsparameter wenigstens Zusammenfügungspunkt-Markierungsinformationen über die Ursprungsbilder umfassen,
wobei das röntgenbasierte abtastende Abbildungssystem **dadurch gekennzeichnet ist, dass** es ein abtastendes Abbildungssystem auf der Basis von Spaltröntgenstrahlen ist, das Spaltradiographie-Röntgentechnologie verwendet und Folgendes umfasst:
ein Positionssteuermodul (2), das zum Steuern von Röntgenstrahlen ausgebildet ist, die von einem Strahlemissionsfenster (322) an beliebiger Position emittiert werden, um von einem voreingestellten Spaltinteressensbereich (422) genau und vollständig empfangen zu werden;
wobei sich das Strahlemissionsfenster (322) und der voreingestellte Spaltinteressensbereich (422) an einer Ausgangsposition des abzubildenden Objekts befinden;
wobei das Positionssteuermodul (2) Folgendes umfasst:
eine Positionssynchronisationseinheit (21), die eine Steuerung umfasst, die zum Steuern von Positionen des Strahlemissionsfensters (322) und des voreingestellten Spaltinteressensbereichs (422) ausgebildet ist;
eine Positionskalibrierungseinheit (22), die zum Positionieren und Kalibrieren des Strahlemissionsfensters (322) und des voreingestellten Spaltinteressensbereichs (422) ausgebildet ist; und
eine Positionsverfolgungseinheit (23), die zum Aktualisieren von Positionen des Strahlemissionsfensters (322) und des voreingestellten Spaltinteressensbereichs (422) ausgebildet ist;
wobei die Positionssynchronisationseinheit (21) und die Positionskalibrierungseinheit (22) auf die Positionsverfolgungseinheit (23) ansprechen.

2. Abtastendes Abbildungssystem auf der Basis von Spaltröntgenstrahlen nach Anspruch 1, ferner umfassend: ein Systeminitialisierungsmodul (1), das dazu ausgebildet ist, das abtastende Abbildungssystem zu initialisieren, wobei das Systeminitialisierungsmodul (1) Folgendes umfasst:
eine Moduserfassungseinheit (11), die zum Erfassen eines Abbildungsdatensatzes des abzubildenden Objekts ausgebildet ist;
eine Parameterübergabeeinheit (12), die zum Übergeben des Abbildungsdatensatzes an das abtastende Abbildungssystem ausgebildet ist; und
eine Ausgangspositions-Reaktionseinheit (13), die dazu ausgebildet ist, als Reaktion auf den Abbildungsdatensatz das Strahlemissionsfenster (322) und den voreingestellten Spaltinteressensbereich (422) so zu steuern, dass sie sich jeweils in einer Systemausgangsposition befinden.

3. Abtastendes Abbildungssystem auf der Basis von Spaltröntgenstrahlen nach Anspruch 1, wobei die Positionskalibrierungseinheit (22) Folgendes umfasst:
eine Untereinheit (221) zur Kalibrierung einer Ausgangsposition, die eine erste optische Anzeigevorrichtung umfasst, die auf die Ausgangsposition des abzubildenden Objekts entlang des Strahlemissionsfensters (322) zeigt und zum Positionieren und Kalibrieren der Ausgangsposition des abzubildenden Objekts, auf die das Strahlemissionsfenster (322) zeigt, und des voreingestellten Spaltinteressensbereichs (422) ausgebildet ist;
eine Untereinheit (222) zur Kalibrierung einer hinteren Endposition, die eine zweite optische Anzeigevorrichtung umfasst, die zum Erzeugen eines Anzeigesignals, das auf eine hintere Endposition des abzubildenden Objekts zeigt, und Durchführen von Positionierung und Kalibrierung an der hinteren Endposition des abzubildenden Objekts, auf die das Strahlemissionsfenster (322) zeigt, und dem voreingestellten Spaltinteressensbereich (422) gemäß dem Anzeigesignal ausgebildet ist; und
eine Untereinheit (223) zur Kalibrierung einer relativen Position, die eine Positionseinstellvorrichtung umfasst, die zur Positionierung und Kalibrierung relativer Positionen des Strahlemissionsfensters (322) und des voreingestellten Spaltinteressensbereichs (422) ausgebildet ist.

4. Abtastendes Abbildungssystem auf der Basis von Spaltröntgenstrahlen nach Anspruch 3, wobei die Positionsverfolgungseinheit (23) dazu ausgebildet ist, Positionen des Strahlemissionsfensters (322) und des voreingestellten Spaltinteressensbereichs (422) mittels Raumkoordinaten-Positionsinformationen zu verfolgen.

5. Abtastendes Abbildungssystem auf der Basis von Spaltröntgenstrahlen nach Anspruch 1, wobei das Emissionsmodul (3) Folgendes umfasst:
eine Kollimationseinheit (31), die zum Kollimieren der Röntgenstrahlen ausgebildet ist, damit sie die Breite des Strahlemissionsfensters (322) aufweisen; und
eine Emissionssteuereinheit (32), die zum Steuern eines Emissionsintervalls der Röntgenstrahlen ausgebildet ist.

6. Abtastendes Abbildungssystem auf der Basis von Spaltröntgenstrahlen nach Anspruch 1, wobei das Abbildungsmodul (4) ferner Folgendes umfasst:
ein Ursprungsbildspeicherungs-Untermodul (41), das ausgebildet ist zum Speichern der Ursprungsbilder des abzubildenden Objekts und der Zusammenfügungsparameter der Ursprungsbilder, die vom voreingestellten Spaltinteressensbereich (422) empfangen werden, falls der voreingestellte Spaltinteressensbereich (422) den an einer beliebigen Position des abzubildenden Objekts abgeschwächten Röntgenstrahl empfängt; und
ein Ursprungsbildübertragungs-Untermodul (42), das zum Übertragen der Ursprungsbilder, die von dem voreingestellten Spaltinteressensbereich (422) erzeugt werden, und der Zusammenfügungsparameter der Ursprungsbilder ausgebildet ist.

7. Abtastendes Abbildungssystem auf der Basis von Spaltröntgenstrahlen nach Anspruch 6, wobei das Ursprungsbildspeicherungs-Untermodul (41) ferner Folgendes umfasst:
eine Strahlempfangseinheit (4110), die zum Empfangen der nach dem Durchdringen des abzubildenden Objekts abgeschwächten Röntgenstrahlen ausgebildet ist;
eine Bilderzeugungseinheit (4111), die zum Erzeugen der Ursprungsbilder des abzubildenden Objekts, die abgeschwächten Röntgenstrahlen entsprechen, ausgebildet ist;
und
eine Bildmarkierungseinheit (4112), die zum Markieren der Zusammenfügungsparameter der Ursprungsbilder ausgebildet ist.

8. Abtastendes Abbildungssystem auf der Basis von Spaltröntgenstrahlen nach Anspruch 7, wobei das Ursprungsbildübertragungs-Untermodul (42) ferner Folgendes umfasst:
eine Ursprungsbild-Sammeleinheit (4210), die zum Sammeln der Ursprungsbilder des Bildspeicherungs-Untermoduls (41) und der Zusammenfügungsparameter der Ursprungsbilder gemäß Positionsbahnen des Strahlemissionsfenster (322) und des voreingestellten Spaltinteressensbereichs (422) ausgebildet ist; und
eine Ursprungsbild-Ausgabeeinheit (4220), die zum Ausgeben der Ursprungsbilder und der Zusammenfügungsparameter der Ursprungsbilder, die von der Ursprungsbild-Sammeleinheit (4210) gesammelt werden, an das Bildrekonstruktionsmodul (5) ausgebildet ist.

9. Abtastendes Abbildungssystem auf der Basis von Spaltröntgenstrahlen nach Anspruch 8, wobei das Bildrekonstruktionsmodul (5) dazu ausgebildet ist, die Ursprungsbilder gemäß den Zusammenfügungsparametern, die von der Bildausgabeeinheit (4220) ausgegeben werden, zu rekonstruieren, um ein vollständiges Bild zu bilden, falls die Bildausgabeeinheit (4220) die Ursprungsbilder an der hinteren Endposition des abzubildenden Objekts ausgibt, wobei das Bildrekonstruktionsmodul (5) Folgendes umfasst:
eine Adressiereinheit (51), die zum Suchen nach den Ursprungsbildern ausgebildet ist, die basierend auf den Zusammenfügungsparametern der Ursprungsbilder zu rekonstruieren sind, die einzeln adressiert werden können; und
eine Zusammenfügungseinheit (52), die ausgebildet ist zum Einstellen, gemäß den Zusammenfügungsparametern, einer Zusammenfügung einer hinteren Endposition einer vorigen Abbildung und einer Anfangsposition einer nächsten Abbildung der Ursprungsbilder, die zu rekonstruieren sind, um ein vollständiges Bild zu bilden.

10. Röntgenbasiertes abtastendes Abbildungsverfahren umfassend:
Emittieren (300) von Röntgenstrahlen mit einer Breite eines Strahlemissionsfensters (322) zu einem abzubildenden Objekt in dem Fall;
Erzeugen (400) von Ursprungsbildern basierend auf empfangenen Röntgenstrahlen, die das abzubildende Objekt durchdringen, wobei wenigstens ein überlappender Bereich zwischen benachbarten Bildern der Ursprungsbilder vorhanden ist; und
Rekonstruieren (500) der Ursprungsbilder, um gemäß Zusammenfügungsparametern der Ursprungsbilder ein vollständiges Bild zu bilden, wobei die Zusammenfügungsparameter wenigstens Zusammenfügungspunkt-Markierungsinformationen über die Ursprungsbilder umfassen;
wobei das röntgenbasierte abtastende Abbildungsverfahren **dadurch gekennzeichnet ist, dass** es ein abtastendes Abbildungsverfahren auf der Basis von Spaltröntgenstrahlen ist, das Spaltradiographie-Röntgentechnologie verwendet und Folgendes umfasst:
Steuern (200) von Röntgenstrahlen, die von dem Strahlemissionsfenster (322) an beliebiger Position emittiert werden, um von einem voreingestellten Spaltinteressensbereich (422) genau und vollständig empfangen zu werden;
wobei sich das Strahlemissionsfenster (322) und der voreingestellte Spaltinteressensbereich (422) an einer Ausgangsposition des abzubildenden Objekts befinden;
wobei der Schritt des Steuerns (200) Folgendes umfasst:
Steuern von Positionen des Strahlemissionsfensters (322) und des voreingestellten Spaltinteressensbereichs (422);
Positionieren und Kalibrieren des Strahlemissionsfensters (322) und des voreingestellten Spaltinteressensbereichs (422);
Aktualisieren von Positionen des Strahlemissionsfensters (322) und des voreingestellten Spaltinteressensbereichs (422);
wobei die Positionssynchronisationseinheit (21) und die Positionskalibrierungseinheit (22) auf die Positionsverfolgungseinheit (23) ansprechen.

## Revendications

1. Système d'imagerie à balayage basé sur des rayons X comprenant :
un module d'émission (3) configuré pour émettre des rayons X présentant une largeur d'une fenêtre d'émission de rayons vers un objet à imager ;
un module d'imagerie (4) configuré pour générer des images d'origine sur la base de rayons X reçus traversant an objet à imager, dans lequel au moins une région de chevauchement existe entre des images adjacentes des images d'origine ; et
un module de reconstruction d'image (5) configuré pour reconstruire les images d'origine pour former une image complète en fonction de paramètres d'assemblage des images d'origine, dans lequel les paramètres d'assemblage comprennent au moins des informations de marquage de points d'assemblage relatives aux images d'origine,
le système d'imagerie à balayage basé sur des rayons X étant **caractérisé en ce qu'**il consiste en un système d'imagerie à balayage basé sur des rayons X fendus utilisant une technologie de radiographie aux rayons X fendus et comprenant :
un module de commande de position (2) configuré pour commander des rayons X émis par une fenêtre d'émission de rayons (322) au niveau de n'importe quelle position de façon à être reçus exactement et complètement par une région fendue d'intérêt prédéfinie (422) ;
la fenêtre d'émission de rayons (322) et la région fendue d'intérêt prédéfinie (422) étant situées au niveau d'une position initiale de l'objet à imager ;
dans lequel le module de commande de position (2) comprend :
une unité de synchronisation de position (21) comprenant une unité de commande configurée pour commander des positions de la fenêtre d'émission de rayons (322) et de la région fendue d'intérêt prédéfinie (422) ;
une unité d'étalonnage de position (22) configurée pour positionner et étalonner la fenêtre d'émission de rayons (322) et la région fendue d'intérêt prédéfinie (422) ; et
une unité de suivi de position (23) configurée pour mettre à jour des positions de la fenêtre d'émission de rayons (322) et de la région fendue d'intérêt prédéfinie (422) ;
l'unité de synchronisation de position (21) et l'unité d'étalonnage de position (22) répondant à l'unité de suivi de position (23).

2. Système d'imagerie à balayage basé sur des rayons X fendus selon la revendication 1, comprenant en outre : un module d'initialisation de système (1) configuré pour initialiser le système d'imagerie à balayage, dans lequel le module d'initialisation de système (1) comprend :
une unité d'acquisition de mode (11) configurée pour acquérir un ensemble de données d'imagerie de l'objet à imager ;
une unité de transfert de paramètre (12) configurée pour transférer l'ensemble de données d'imagerie au système d'imagerie à balayage ; et
une unité de réponse de position initiale (13) configurée pour commander, en réponse à l'ensemble de données d'imagerie, la fenêtre d'émission de rayons (322) et la région fendue d'intérêt prédéfinie (422) pour être respectivement situées en une position initiale de système.

3. Système d'imagerie à balayage basé sur des rayons X fendus selon la revendication 1, dans lequel l'unité d'étalonnage de position (22) comprend :
une sous-unité d'étalonnage de position initiale (221) comprenant un premier dispositif d'indication optique pointant vers la position initiale de l'objet à imager le long de la fenêtre d'émission de rayons (322) et configuré pour positionner et étalonner la position initiale de l'objet à imager vers lequel pointent la fenêtre d'émission de rayons (322) et la région fendue d'intérêt prédéfinie (422) ;
une sous-unité d'étalonnage de position d'extrémité de queue (222) comprenant un deuxième dispositif d'indication optique configuré pour générer un signal d'indication pointant vers une position d'extrémité de queue de l'objet à imager, et effectuer un positionnement et un étalonnage sur la position d'extrémité de queue de l'objet à imager vers lequel pointent la fenêtre d'émission de rayons (322) et la région fendue d'intérêt prédéfinie (422) en fonction du signal d'indication ; et
une sous-unité d'étalonnage de position relative (223) comprenant un dispositif de réglage de position configuré pour positionner et étalonner des positions relatives de la fenêtre d'émission de rayons (322) et de la région fendue d'intérêt prédéfinie (422).

4. Système d'imagerie à balayage basé sur des rayons X fendus selon la revendication 3, dans lequel l'unité de suivi de position (23) est configurée pour suivre des positions de la fenêtre d'émission de rayons (322) et de la région fendue d'intérêt prédéfinie (422) au moyen d'informations de position de coordonnées spatiales.

5. Système d'imagerie à balayage basé sur des rayons X fendus selon la revendication 1, dans lequel le module d'émission (3) comprend :
une unité de collimation (31) configurée pour collimater les rayons X de façon à avoir la largeur de la fenêtre d'émission de rayons (322) ; et
une unité de commande d'émission (32) configurée pour commander un intervalle d'émission des rayons X.

6. Système d'imagerie à balayage basé sur des rayons X fendus selon la revendication 1, dans lequel le module d'imagerie (4) comprend en outre :
un sous-module de stockage d'images d'origine (41) configuré pour stocker les images d'origine de l'objet à imager et les paramètres d'assemblage des images d'origine reçues par la région fendue d'intérêt prédéfinie (422) dans le cas où la région fendue d'intérêt prédéfinie (422) reçoit les rayons X atténués au niveau de n'importe quelle position de l'objet à imager ; et
un sous-module de transmission d'images d'origine (42) configuré pour transmettre les images d'origine générées par la région fendue d'intérêt prédéfinie (422) et les paramètres d'assemblage des images d'origine.

7. Système d'imagerie à balayage basé sur des rayons X fendus selon la revendication 6, dans lequel le sous-module de stockage d'images d'origine (41) comprend en outre :
une unité de réception de rayons (4110) configurée pour recevoir les rayons X atténués après avoir traversé l'objet à imager ;
une unité de génération d'images (4111) configurée pour générer les images d'origine de l'objet à imager correspondant à des rayons X atténués ; et
une unité de marquage d'images (4112) configurée pour marquer les paramètres d'assemblage des images d'origine.

8. Système d'imagerie à balayage basé sur des rayons X fendus selon la revendication 7, dans lequel le sous-module de transmission d'images d'origine (42) comprend en outre :
une unité de collecte d'images d'origine (4210) configurée pour collecter les images d'origine du sous-module de stockage d'images (41) et les paramètres d'assemblage des images d'origine en fonction de trajectoires de position de la fenêtre d'émission de rayons (322) et de la région fendue d'intérêt prédéfinie (422) ; et
une unité de délivrance d'images d'origine (4220) configurée pour délivrer en sortie les images d'origine et les paramètres d'assemblage des images d'origine collectées par l'unité de collecte d'images d'origine (4210) au module de reconstruction d'image (5).

9. Système d'imagerie à balayage basé sur des rayons X fendus selon la revendication 8, dans lequel le module de reconstruction d'image (5) est configuré pour reconstruire les images d'origine en fonction des paramètres d'assemblage délivrés en sortie par le module de délivrance d'images (4220) pour former une image complète dans le cas où le module de délivrance d'images (4220) délivre en sortie les images d'origine au niveau de la position d'extrémité de queue de l'objet à imager, dans lequel le module de reconstruction d'image (5) comprend :
une unité d'adressage (51) configurée pour rechercher les images d'origine à reconstruire sur la base des paramètres d'assemblage des images d'origine qui peuvent être adressées une par une ; et
une unité d'assemblage (52) configurée pour ajuster, en fonction des paramètres d'assemblage, un assemblage d'une position d'extrémité de queue d'une imagerie précédente et d'une position de départ d'une imagerie suivante des images d'origine à reconstruire pour former une image complète.

10. Procédé d'imagerie par balayage basé sur des rayons X comprenant :
l'émission (300) de rayons X présentant une largeur d'une fenêtre d'émission de rayons (322) vers un objet à imager ;
la génération (400) d'images d'origine sur la base de rayons X reçus traversant l'objet à imager, dans lequel au moins une région de chevauchement existe entre des images adjacentes des images d'origine ; et
la reconstruction (500) des images d'origine pour former une image complète en fonction de paramètres d'assemblage des images d'origine, dans lequel les paramètres d'assemblage comprennent au moins des informations de marquage de points d'assemblage relatives aux images d'origine ;
le procédé d'imagerie à balayage basé sur des rayons X étant **caractérisé en ce qu'**il consiste en un procédé d'imagerie à balayage basé sur des rayons X fendus utilisant une technologie de radiographie aux rayons X fendus et comprenant :
la commande (200) de rayons X émis par la fenêtre d'émission de rayons (322) au niveau de n'importe quelle position de façon à être reçus exactement et complètement par une région fendue d'intérêt prédéfinie (422) ;
la fenêtre d'émission de rayons (322) et la région fendue d'intérêt prédéfinie (422) étant situées au niveau d'une position initiale de l'objet à imager ;
dans lequel l'étape de commande (200) comprend :
la commande de positions de la fenêtre d'émission de rayons (322) et de la région fendue d'intérêt prédéfinie (422) ;
le positionnement et l'étalonnage de la fenêtre d'émission de rayons (322) et de la région fendue d'intérêt prédéfinie (422) ;
la mise à jour de positions de la fenêtre d'émission de rayons (322) et de la région fendue d'intérêt prédéfinie (422) ;
l'unité de synchronisation de position (21) et l'unité d'étalonnage de position (22) répondant à l'unité de suivi de position (23).
